# EUROPEAN PATENT APPLICATION

(11) **EP 3 571 996 A1**
(43) Date of publication of application: **27.11.2019**
(21) Application number: 19162191.1
(22) Date of filing: 12.03.2019
(51) Int. Cl.: A61B 5/20

(54) **URODYNAMIC MONITORING SYSTEM AND DRAINAGE MONITORING CONTROL UNIT THEREOF**

(30) Priority: 25.05.2018 CN 201810517441
(71) Applicant: Zhejiang Lexin Medical Technology Co., Ltd., Jiaxing, Zhejiang (CN)
(72) Inventor: LYU, Baicheng, Jiaxing, Zhejiang (CN); CHEN, Junjie, Jiaxing, Zhejiang (CN); FU, Fei, Jiaxing, Zhejiang (CN); LI, Guorong, Jiaxing, Zhejiang (CN)
(74) Representative: Schmid, Nils T.F.

(57) **Abstract**

The present invention relates to a urodynamic monitoring system and a drainage monitoring control unit thereof. The present invention includes a drainage monitoring control unit and a monitoring host that is provided with a mainboard, a control component, and a weighing component, where the drainage monitoring control unit is formed by sequentially connecting a front drainage pipeline, a monitoring controller, a rear drainage pipeline, a urine collection bag, and the front drainage pipeline is provided with a first three-way valve communicated with air. According to the present invention, the first three-way valve communicated with air may be disposed on the front drainage pipeline, air pressure on a bladder is measured by switching a working state of the first three-way valve, and an accurate value of intravesical pressure is obtained by subtracting the air pressure on the bladder from pressure of urine in a monitoring control cavity, so as to resolve a technical problem in the prior that bionic urine control cannot be implemented and intra-abdominal pressure cannot be continuously and dynamically reflected because intravesical pressure cannot be accurately monitored.

## Description

This application claims priority to Chinese application number 201810517441.7, filed May 25 2018, with a title of URODYNAMIC MONITORING SYSTEM AND DRAINAGE MONITORING CONTROL UNIT THEREOF. The above-mentioned patent application is incorporated herein by reference in its entirety.

### TECHNICAL FIELD

The present invention relates to a urodynamic monitoring system and a drainage monitoring control unit thereof, and belongs to the field of medical appliances.

### BACKGROUND

Intra-abdominal pressure (IAP) is pressure within an abdominal cavity and is one of important physiological parameters in clinical diagnosis and disease treatment, and intravesical pressure measurement is a gold standard for indirectly measuring intra-abdominal pressure.

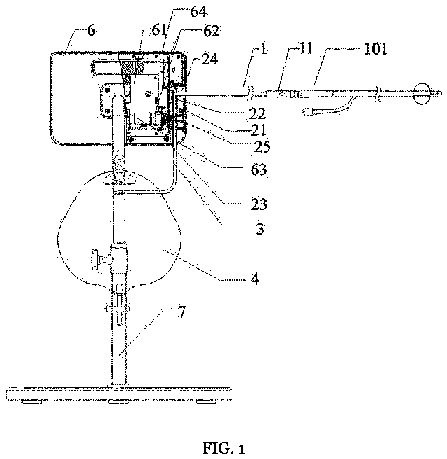

Clinically, catheterizing a patient via an indwelling urinary catheter inserted into a urethra is a widely applied normal operation currently. To avoid degeneration of bladder distension and contraction functions caused due to continuous discharge of urine from a bladder of a patient, a technical person researches and developments a urodynamic monitoring system capable of preventing degeneration of bladder distension and contraction functions. The urodynamic monitoring system includes a monitoring host and a drainage monitoring control unit (as shown in FIG. 1), where the drainage monitoring control unit is provided with a front drainage pipeline 1 connected to an indwelling urinary catheter 101, a monitoring controller 2, a rear drainage pipeline 3, and a urine collection bag 4; the monitoring controller 2 is provided with a monitoring control cavity 21, a monitoring component 22, and a control valve 25; the monitoring host 6 is provided with a mainboard 61, a control component 62, and a weighing component 63 that are accommodated inside a housing 64; and the monitoring host 6 is fastened to an adjusting bracket 7. When the urodynamic monitoring system drains urine of a patient, urine in a bladder outflows through the indwelling urinary catheter 101, and flows into the monitoring control cavity 21 through the front drainage pipeline 1. In this case, the control valve 25 blocks flow from a urine outlet 23 and a urine inlet 24 of the monitoring control cavity 21; pressure of urine in the monitoring control cavity 21 continually increases with an increase of pressure of urine in the bladder; the monitoring component 22 monitors pressure of urine in the monitoring control cavity 21 and outputs pressure of urine to the mainboard 61; and the mainboard 61 receives a pressure signal output by the monitoring component 22. When pressure reaches or exceeds a urination threshold, and duration is greater than or equal to a preset time, the mainboard 61 sends a urination instruction to the control component 62 to make the urine outlet 23 communicated with the urine inlet 24 via the control valve 25; the urine is guided into the urine collection bag 4 through the rear drainage pipeline 3 after passing through the urine outlet 23; and the weighing component 63 continuously measures a weight of the urine in the urine collection bag 4 and provides urine volume data to the mainboard 61.

In the foregoing urodynamic monitoring system, the monitoring host 6 is used to control, based on intravesical pressure via the control valve 25, whether to perform bladder urination. This resolves a problem of degeneration of bladder distension and contraction functions caused due to continuous urine discharge of urine from a bladder of a patient. However, there are still the following disadvantages in actual application.
1. The foregoing urodynamic monitoring system cannot accurately monitor intravesical pressure. A reason is that when the monitoring control cavity 21 is communicated with a bladder, a pressure signal output by the monitoring component 22 is intravesical pressure including air pressure on the bladder. The mainboard 61 receives the pressure signal output by the monitoring component 22. Pressure indicated by the pressure signal includes the air pressure on the bladder, that is, there is an error between the intravesical pressure and the pressure indicated by the pressure signal output by the monitoring component 22, therefore when the pressure indicated by the pressure signal is greater than or equal to a urination threshold, the intravesical pressure of the patient does not reach the urination threshold of normal physiological urination of a human body. In a case in which the air pressure on the bladder cannot be monitored and an error caused by the air pressure cannot be eliminated, the existing urodynamic monitoring system can neither accurately monitor intravesical pressure nor truly implement bionic urine control.
2. In the foregoing existing urodynamic monitoring system, when a patient requires bladder irrigation, a connector 11 needs to be separated from the indwelling urinary catheter 101, so that the indwelling urinary catheter 101 is connected to a transfusion perfusion pipeline; after the bladder irrigation operation is completed, the indwelling urinary catheter 101 is separated from the transfusion perfusion pipeline; and the indwelling urinary catheter 101 is reconnected to the urodynamic monitoring system through the connector 11. The connector 11 is repeatedly inserted into or removed from the indwelling urinary catheter 101. In this case, not only the operation is relatively complex, but also it is easy to cause bacterial contamination on the connector 11 and the indwelling urinary catheter 101, thereby bringing a risk of bacterial infection to a patient.
3. When the foregoing existing urodynamic monitoring system is used, the urine collection bag 4 is hung in open space below the monitoring host 6, and is easy to shake due to touch of nurses and caretakers moving frequently beside a sickbed, so as to interfere with weighing data and affect accuracy of monitoring data of related urodynamic parameters.
4. Because intravesical pressure cannot be continuously dynamically accurately monitored, the existing urodynamic monitoring systems cannot continuously dynamically accurately reflect intra-abdominal pressure (IAP).

### SUMMARY

To mainly resolve a technical defect in the prior art that bionic urine control cannot be implemented and intra-abdominal pressure cannot be continuously and dynamically reflected because intravesical pressure cannot be accurately monitored, the present invention provides a drainage monitoring control unit that can obtain accurate monitoring data of intravesical pressure to implement bionic urine control and that can continuously and dynamically monitor intravesical pressure to indirectly implement intra-abdominal pressure monitoring.

The present invention mainly resolves the foregoing technical defect by using the following technical solution. The present invention includes a front drainage pipeline, a monitoring controller, a rear drainage pipeline, and a urine collection bag, where the monitoring controller includes a monitoring control cavity that is provided with a urine outlet communicated with the urine collection bag via the rear drainage pipeline and that is provided with a urine inlet connected to the front drainage pipeline, a monitoring component that monitors pressure of urine in the monitoring control cavity and outputs a pressure signal, and a control valve that controls communication or blocking between the urine inlet and the urine outlet; the front drainage pipeline includes a connector connected to an indwelling urinary catheter, and further includes a first three-way valve, where the first three-way valve is provided with an air inlet communicated with air, a first inlet communicated with the connector, and a first outlet connected to the urine inlet; the first three-way valve can switch between two working states: blocking the first inlet but making the air inlet communicate with the first outlet and blocking the air inlet but making the first inlet communicate with the first outlet; and a drift diameter of the first three-way valve is less than an inner diameter of the indwelling urinary catheter.

Preferably, the front drainage pipeline further includes a second three-way valve, where the second three-way valve is connected between the connector and the first three-way valve, and includes a perfusion port that can be connected to an external infusion pipeline, a second inlet connected to the connector, and a second outlet connected to the first inlet through a pipeline connected between the first three-way valve and the second three-way valve; the second three-way valve can switch between two working states: blocking the second outlet but making the perfusion port communicate with the second inlet and blocking the perfusion port but making the second inlet communicate with the second outlet; and a drift diameter of the second three-way valve is not less than the inner diameter of the indwelling urinary catheter.

Preferably, the monitoring controller further includes an electronic tag, and the electronic tag is configured to: determine a correspondence between a patient and the monitoring controller, store data information about use of the monitoring controller by the patient, and limit service life of the monitoring controller.

To mainly resolve the technical defect in the prior art that bionic urine control cannot be implemented and intra-abdominal pressure cannot be continuously and dynamically reflected because intravesical pressure cannot be accurately monitored, the present invention further provides a urodynamic monitoring system for more accurate urodynamic parameter monitoring.

The present invention mainly resolves the foregoing technical defect by using the following technical solution. The present invention includes a drainage monitoring control unit and a monitoring host that is provided with a mainboard, a control component, and a weighing component, where the weighing component is configured to continuously measure a weight of urine in the urine collection bag and output a weight signal to the mainboard; the drainage monitoring control unit is the drainage monitoring control unit according to claims 1 to 3; the mainboard receives a pressure signal output by the monitoring component; and when a pressure value that is obtained by subtracting air pressure at a position of a patient from pressure of urine in the monitoring control cavity is greater than or equal to a urination threshold, and duration reaches or exceeds a preset time, the mainboard outputs an instruction of making the urine inlet communicated with the urine outlet to the control component, and the control component drives a valve element of the control valve to rotate to make the urine inlet communicated with the urine outlet.

Preferably, the monitoring host further includes a housing, where the drainage monitoring control unit, the mainboard, the control component, and the weighing component are accommodated inside the housing.

Preferably, the urodynamic monitoring system further includes a laser level indicator, where the laser level indicator is fastened on the housing, is located at a same horizontal height as the monitoring component, and can emit horizontal indicating light.

Preferably, the urodynamic monitoring system further includes an adjusting bracket, where the adjusting bracket is provided with an annular anti-interference frame; the anti-interference frame is located below the monitoring host; and the urine collection bag is hung on the adjusting bracket, and is located in a hollow area of the anti-interference frame when being weighed via the weighing component.

Therefore, the present invention has a novel design, an appropriate structure, and the following advantages.

In the present invention, the front drainage pipeline in the drainage monitoring control unit is provided with the first three-way valve that can be communicated with air. When the monitoring control cavity and a bladder has a same height and a pipeline is fully filled with liquid, when the first three-way valve is in a working state of opening the air inlet to communicate with air and the first outlet and closing the first inlet, pressure generated by liquid in the monitoring control cavity is equal to air pressure, and equal to air pressure on a bladder; when the first three-way valve is in a working state of closing the air inlet to block air and making the first inlet communicate with the first outlet, pressure generated by liquid in the monitoring control cavity is equal to a sum of intravesical pressure and the air pressure on the bladder; the monitoring component monitors pressure generated by urine in the monitoring control cavity and outputs a pressure signal; the mainboard receives the pressure signal output by the monitoring component; and the mainboard can obtain an accurate intravesical pressure value by subtracting a value of the pressure generated by the urine in the monitoring control cavity when the first three-way valve is in the working state of opening the air inlet to be communicate with the first outlet and closing the first inlet, from a value of the pressure generated by the urine in the monitoring control cavity when the first three-way valve is in the working state of closing the air inlet to block air and making the first inlet communicate with the first outlet. Therefore, a technical problem in the prior that a monitoring host cannot obtain accurate intravesical pressure because air pressure on a bladder cannot be monitored and therefore bionic urine control cannot be implemented is resolved, and technical effects of bladder distension and contraction functions during retention catheterization are remained. In addition, intravesical pressure can be continuously, dynamically, and accurately monitored and therefore intra-abdominal pressure (IAP) can be continuously, dynamically, and accurately reflected. In the present invention, a drift diameter of the first three-way valve is not less than an inner diameter of the indwelling urinary catheter, so that a flow rate of urine in the drainage pipeline is not affected, and interference of the first three-way valve on urinary flow rate data of bladder intubation can be avoided.

In the present invention, the front drainage pipeline in the drainage monitoring control unit is further provided with the second three-way valve, the perfusion port of the second three-way valve can be connected to the external infusion pipeline. Through switching of working states of the second three-way valve, two working states can be switched on the front drainage pipeline during urine drainage control and bladder irrigation without inserting the connector into or removing the connector from the front drainage pipeline, so as to avoid a risk of bacterial contamination resulting from repeatedly inserting a connector into or removing a connector from the indwelling urinary catheter. In the present invention, a drift diameter of the second three-way valve is not less than the inner diameter of the indwelling urinary catheter, so that a flow rate of urine in the drainage pipeline is not affected, and interference of the second three-way valve on urinary flow rate data of bladder intubation can be avoided.

In the present invention, because the urodynamic monitoring system is provided with the laser level indicator that is located at a same horizontal height as the monitoring component, when light emitted by the laser level indicator is indicated at a midaxillary line of a patient, the monitoring component can be located accurately at a same horizontal height as the bladder of the patient. This can effectively avoid an error in monitoring data of intravesical pressure resulting from a height difference between the monitoring component and the bladder of the patient.

In the present invention, because the urodynamic monitoring system is provided with the adjusting bracket with an anti-interference frame, when the urine collection bag is located in a hollow area of the anti-interference frame when being hung below the monitoring host for urine weighing, blocking of the anti-interference frame avoids a case in which interference is caused to weighing data because the urine collection bag is shaken by unintentional touch of nurses or caretakers moving frequently beside a sickbed and consequently an error is caused to all data related to the weighing data.

### BRIEF DESCRIPTION OF THE DRAWINGS

FIG. 1 is a schematic diagram of the prior art;
FIG. 2 is a schematic diagram of an embodiment of a drainage monitoring control unit according to the present invention;
FIG. 3 is a schematic diagram of an embodiment of another drainage monitoring control unit according to the present invention; and
FIG. 4 is a schematic diagram of an embodiment of a urodynamic monitoring system according to the present invention.

Reference signs: 1, front drainage pipeline; 11, connector; 12, first three-way valve; 121, air inlet; 122, first inlet; 123, first outlet; 13, second three-way valve; 131, perfusion port; 132, second inlet; 133, second outlet; 2, monitoring controller; 21, monitoring control cavity; 22, monitoring component; 23, urine outlet; 24, urine inlet; 25, control valve; 3, rear drainage pipeline; 4, urine collection bag; 5, electronic tag; 6, monitoring host; 7, adjusting bracket; 8, drainage monitoring control unit; 9, laser level indicator.

### DETAILED DESCRIPTION

The following further specifically describes technical solutions of the present invention with reference to accompanying drawings by using the embodiments.

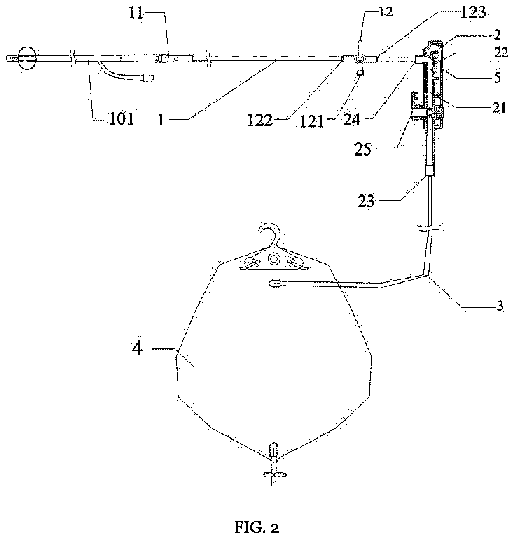

Embodiment 1: As shown in FIG. 2, the present invention includes a front drainage pipeline 1, a monitoring controller 2, a rear drainage pipeline 3, a urine collection bag 4, and an electronic tag 5.

The monitoring controller 2 includes a monitoring component 22, a control valve 25, and a monitoring control cavity 21 that is provided with a urine outlet 23 communicated with the urine collection bag 4 via the rear drainage pipeline 3 and that is provided with a urine inlet 24 connected to the front drainage pipeline 1, where the monitoring component 22 monitors pressure of urine in the monitoring control cavity 21 and outputs a pressure signal, and the control valve 25 controls communication or blocking between the urine inlet 24 and the urine outlet 23.

The front drainage pipeline 1 includes a connector 11 connected to an indwelling urinary catheter 101 and a first three-way valve 12.

The first three-way valve 12 is provided with an air inlet 121 communicated with air, a first inlet 122 communicated with the connector 11, and a first outlet 123 connected to the urine inlet 24; the first three-way valve 12 can switch between two working states: blocking the first inlet 122 but making the air inlet 121 communicate with the first outlet 123 and blocking the air inlet 121 but making the first inlet 122 communicate with the first outlet 123; and a drift diameter of the first three-way valve 12 is not less than an inner diameter of the indwelling urinary catheter 101.

The electronic tag 5 is fastened to the monitoring controller 2, and is configured to: determine a correspondence between a patient and the monitoring controller 2, store data information about use of the monitoring controller 2 by the patient, and limit service life of the monitoring controller 2.

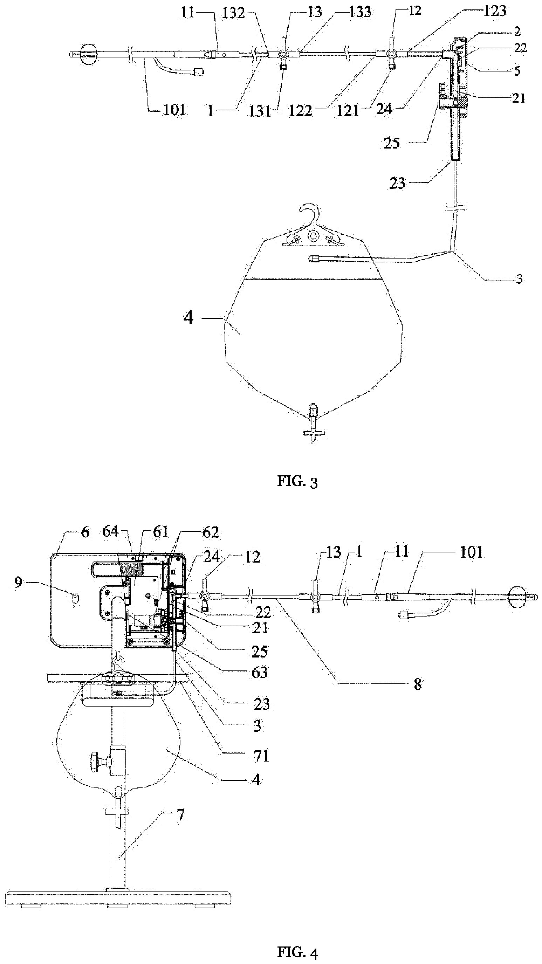

Embodiment 2: As shown in FIG. 3, the present invention includes a front drainage pipeline 1, a monitoring controller 2, a rear drainage pipeline 3, a urine collection bag 4, and an electronic tag 5.

The monitoring controller 2 includes a monitoring component 22, a control valve 25, and a monitoring control cavity 21 that is provided with a urine outlet 23 communicated with the urine collection bag 4 via the rear drainage pipeline 3 and that is provided with a urine inlet 24 connected to the front drainage pipeline 1, where the monitoring component 22 monitors pressure of urine in the monitoring control cavity 21 and outputs a pressure signal, and the control valve 25 controls communication or blocking between the urine inlet 24 and the urine outlet 23.

The front drainage pipeline 1 includes a connector 11 connected to an indwelling urinary catheter 101, a first three-way valve 12, and a second three-way valve 13.

The first three-way valve 12 is provided with an air inlet 121 communicated with air, a first inlet 122, and a first outlet 123 connected to the urine inlet 24; the first three-way valve 12 can switch between two working states: blocking the first inlet 122 but making the air inlet 121 communicate with the first outlet 123 and blocking the air inlet 121 but making the first inlet 122 communicate with the first outlet 123; and a drift diameter of the first three-way valve 12 is not less than an inner diameter of the indwelling urinary catheter 101.

The second three-way valve 13 includes a perfusion port 131 that can be connected to an external infusion pipeline, a second inlet 132 connected to the connector 11, and a second outlet 133 connected to the first inlet 122 through a pipeline connected between the first three-way valve 12 and the second three-way valve 13; the second three-way valve 13 can switch between two working states: blocking the second outlet 133 but making the perfusion port 131 communicate with the second inlet 132 and blocking the perfusion port 131 but making the second inlet 132 communicate with the second outlet 133; and a drift diameter of the second three-way valve 13 is not less than the inner diameter of the indwelling urinary catheter 101.

The electronic tag 5 is fastened to the monitoring controller 2, and is configured to: determine a correspondence between a patient and the monitoring controller 2, store data information about use of the monitoring controller 2 by the patient, and limit service life of the monitoring controller 2.

Embodiment 3: as shown in FIG. 3 and FIG. 4, the present invention includes a monitoring host 6 including a mainboard 61, a control component 62, a weighing component 63, and a housing 64; an adjusting bracket 7 configured to adjust a height of the monitoring host 6; a drainage monitoring control unit 8; and a laser level indicator 9.

The mainboard 61, the control component 62, the weighing component 63, and the drainage monitoring control unit 8 are accommodated inside the housing 64, where the mainboard 61 receives a pressure signal output by a monitoring component 22, and obtain air pressure at a position of a patient based on pressure of urine in the monitoring control cavity 21 in two working states: blocking the urine at the air inlet 121 and making the urine flow through the air inlet 121; and when a pressure value obtained by subtracting the air pressure at the position of the patient from pressure of urine in the monitoring control cavity 21 is greater than or equal to a urination threshold, and duration reaches or exceeds a preset time, the mainboard 61 outputs an instruction of making the urine inlet 24 communicated with the urine outlet 23, and the control component 62 drives a valve element of the control valve 25 to rotate to make the urine inlet 24 communicated with the urine outlet 23.

The laser level indicator 9 is fastened on the housing 64, and is located at a same horizontal height as the monitoring component 22, and the laser level indicator 9 can emit horizontal indicating light.

The adjusting bracket 7 is provided with an annular anti-interference frame 71, where the anti-interference frame 71 is located below the monitoring host 6, and the urine collection bag 4 is hung on the adjusting bracket 7, and is located in a hollow area of the anti-interference frame 71 when being weighed via the weighing component 63.

When the present invention is used, the front drainage pipeline 1 and the rear drainage pipeline 3 pass through the housing 64; the connector 11 is connected to the indwelling urinary catheter 101; the urine collection bag 4 is hung below the monitoring host 6 and is located in a hollow area of the anti-interference frame 71; the adjusting bracket 7 adjusts a height of the monitoring host 6, so that light emitted by the laser level indicator 9 is indicated at a midaxillary line of a patient, to determine that the bladder of the patient is located at a same horizontal height as the monitoring component 22.

During drainage monitoring and control, the second three-way valve 13 is in a working state of blocking the perfusion port 131 but making the second inlet 132 communicate with the second outlet 133; the first three-way valve 12 is in a working state of blocking the air inlet 121 but making the first inlet 122 communicate with the first outlet 123; the valve element of the control valve 25 is rotated to make the urine inlet 24 communicated with the urine outlet 23. In this case, urine in the bladder flows into the monitoring control cavity 21 after passing through the front drainage pipeline 1. After the urine is fully filled in the front drainage pipeline 1 and the monitoring control cavity 21, the valve element of the control valve 25 is rotated to block between the urine inlet 24 and the urine outlet 23, pressure generated by urine in the monitoring control cavity 21 gradually increases with an increase of pressure of urine in the bladder; and the monitoring component 22 monitors the pressure of the urine in the monitoring control cavity 21 and outputs a pressure signal.

During measurement of air pressure on a bladder of a patient, the first three-way valve 12 first switches to a state of blocking the first inlet 122 but making the air inlet 121 communicate with the first outlet 123. In this case, the front drainage pipeline 1 is in a blocked state because the first inlet 122 is blocked, and the monitoring control cavity 21 is communicated with external air via the air inlet 121 of the first three-way valve 12. In this case, pressure of urine in the monitoring control cavity 21 is equal to the air pressure on the bladder, and the monitoring component 22 outputs a pressure signal to the mainboard 61. The first three-way valve 12 then switches back to the working state of blocking the air inlet 121 but making the first inlet 122 communicate with the first outlet 123, and communication between the bladder and the monitoring control chamber 21 is restored.

The mainboard 61 receives the pressure signal output by the monitoring component 22. When a pressure value that is obtained by subtracting the air pressure on the bladder of the patient from the pressure generated by the urine in the monitoring control cavity 21 is greater than or equal to a urination threshold, and duration reaches or exceeds a preset time, the mainboard 61 outputs an instruction of making the urine inlet 24 communicated with the urine outlet 23 to the control component 62, and the control component 62 drives a valve element of the control valve 25 to rotate to make the urine inlet 24 communicated with the urine outlet 23. The urine in the bladder is guided into the urine collection bag 4 via the indwelling urinary catheter 101 and after passing through the front drainage pipeline 1, the monitoring controller 2, and the rear drainage pipeline 3; and the weighing component 63 continuously measures a weight of the urine in the urine collection bag 4 and outputs a urine weight signal to the mainboard 61.

The pressure value that is obtained by subtracting the air pressure on the bladder of the patient from the pressure generated by the urine in the monitoring control cavity 21 is accurate intravesical pressure, and intra-abdominal pressure (IAP) can be continuously dynamically accurately reflected by continuously dynamically accurately monitoring the intravesical pressure.

When a patient requires bladder irrigation, the perfusion port 131 of the second three-way valve 13 is connected to a perfusion pipeline, the second three-way valve 13 switches to a working state of making the perfusion port 131 communicate with the second inlet 132 and blocking the second outlet 133; perfusion pharmaceutical liquid enters the second three-way valve 13 through the perfusion port 131, and enters the bladder after passing through the second inlet 132, the connector 11, and the indwelling urinary catheter 101. When all the perfusion pharmaceutical liquid is output into the bladder, the second three-way valve 13 switches to a working state of blocking the perfusion port 131 but making the second inlet 132 communicate with the second outlet 133; the mainboard 61 sends an instruction of making the urine inlet 24 communicated with the urine outlet 23 to the control component 62; the control component 62 drives the valve element of the control valve 25 to rotate to make the urine inlet 24 communicated with the urine outlet 23. The perfusion pharmaceutical liquid in the bladder flows into the urine collection bag 4 after successively passing through the indwelling urinary catheter 101, the connector 11, the second three-way valve 13, the first three-way valve 12, the monitoring control cavity 21, and the rear drainage pipeline 3.

The foregoing provides descriptions of the technical solutions, the accompanying drawings, and the embodiments of the present invention to describe the present invention, but the descriptions of the accompanying drawings and the embodiments are only used to explain and help understand the present invention, and shall not constructed as an improper limitation on the present invention. Technical solutions formed by equivalent or same variation and adjustment made by a person skilled in the art based on the technical solutions, the accompanying drawings, or the embodiments of the present invention all fall within the protection scope of the present invention.

## Claims

1. A drainage monitoring control unit, comprising a front drainage pipeline (1), a monitoring controller (2), a rear drainage pipeline (3), and a urine collection bag (4), wherein the monitoring controller (2) comprises a monitoring control cavity (21) that is provided with a urine outlet (23) communicated with the urine collection bag (4) via the rear drainage pipeline (3) and that is provided with a urine inlet (24) connected to the front drainage pipeline (1), a monitoring component (22) that monitors pressure of urine in the monitoring control cavity (21) and outputs a pressure signal, and a control valve (25) that controls communication or blocking between the urine inlet (24) and the urine outlet (23); the front drainage pipeline (1) comprises a connector (11) connected to an indwelling urinary catheter (101), and further comprises a first three-way valve (12), wherein the first three-way valve (12) is provided with an air inlet (121) communicated with air, a first inlet (122) communicated with the connector (11), and a first outlet (123) connected to the urine inlet (24); the first three-way valve (12) can switch between two working states: blocking the first inlet (122) but making the air inlet (121) communicate with the first outlet (123) and blocking the air inlet (121) but making the first inlet (122) communicate with the first outlet (123); and a drift diameter of the first three-way valve (12) is not less than an inner diameter of the indwelling urinary catheter (101).

2. The drainage monitoring control unit according to claim 1, wherein the front drainage pipeline (1) further comprises a second three-way valve (13) connected between the connector (11) and the first three-way valve (12); the second three-way valve (13) is provided with a perfusion port (131) that can be connected to an external infusion pipeline, a second inlet (132) connected to the connector (11), and a second outlet (133) connected to the first inlet (122) through a pipeline connected between the first three-way valve (12) and the second three-way valve (13); the second three-way valve 13 can switch between two working states: blocking the second outlet (133) but making the perfusion port (131) communicate with the second inlet (132) and blocking the perfusion port (131) but making the second inlet (132) communicate with the second outlet (133); and a drift diameter of the second three-way valve (13) is not less than the inner diameter of the indwelling urinary catheter (101).

3. The drainage monitoring control unit according to claim 1 or 2, wherein the monitoring controller (2) further comprises an electronic tag (5), and the electronic tag (5) is configured to: determine a correspondence between a patient and the monitoring controller (2), store data information about use of the monitoring controller (2) by the patient, and limit service life of the monitoring controller (2).

4. A urodynamic monitoring system particularly according to one of the proceeding claims, comprising a drainage monitoring control unit (8) and a monitoring host (6) that is provided with a mainboard (61), a control component (62), and a weighing component (63), wherein the weighing component (63) is configured to continuously measure a weight of urine in a urine collection bag (4) and output a weight signal to the mainboard (61); the drainage monitoring control unit (8) is the drainage monitoring control unit according to claims 1 to 3; the mainboard (61) receives a pressure signal output by a monitoring component (22), and when a pressure value that is obtained by subtracting air pressure at a position of a patient from pressure of urine in a monitoring control cavity (21) is greater than or equal to a urination threshold, and duration reaches or exceeds a preset time, the mainboard (61) outputs an instruction of making a urine inlet (24) communicated with a urine outlet (23) to a control component (62), and the control component (62) drives a valve element of the control valve (25) to rotate to make the urine inlet (24) communicated with the urine outlet (23).

5. The urodynamic monitoring system according to claim 4, wherein the monitoring host (6) further comprises a housing (64); and the drainage monitoring control unit (8), the mainboard (61), the control component (62), and the weighing component (63) are accommodated inside the housing (64).

6. The urodynamic monitoring system according to claim 4 or 5, wherein the urodynamic monitoring system further comprises a laser level indicator (9), and the laser level indicator (9) is located at a same horizontal height as the monitoring component (22), and can emit horizontal indicating light.

7. The urodynamic monitoring system according to claim 4 to 6, wherein the urodynamic monitoring system further comprises a laser level indicator (9), and the laser level indicator (9) is fastened on the housing (64), is located at a same horizontal height as the monitoring component (22), and can emit horizontal indicating light.

8. The urodynamic monitoring system according to claim 4, claim 5, claim 6, or claim 7, wherein the urodynamic monitoring system further comprises an adjusting bracket (7) configured to adjust a height of the monitoring host (6), and the monitoring host (6) is fastened to the adjusting bracket (7).

9. The urodynamic monitoring system according to claim 8, wherein the adjusting bracket (7) is provided with an annular anti-interference frame (71); the anti-interference frame (71) is located below the monitoring host (6); and the urine collection bag (4) is hung on the adjusting bracket (7), and is located in a hollow area of the anti-interference frame (71) when being weighed via the weighing component (63).
